(19) Europäisches Patentamt
European Patent Office
Office européen des brevets

(11) **EP 4 036 556 A1**

(12) **EUROPEAN PATENT APPLICATION**
published in accordance with Art. 153(4) EPC

(43) Date of publication:
**03.08.2022 Bulletin 2022/31**

(21) Application number: **20880536.6**

(22) Date of filing: **02.10.2020**

(51) International Patent Classification (IPC):
**G01N 21/17** (2006.01)  **C12N 5/071** (2010.01)
**C12Q 1/04** (2006.01)

(52) Cooperative Patent Classification (CPC):
**C12Q 1/04; G01N 21/17**

(86) International application number:
**PCT/JP2020/037591**

(87) International publication number:
**WO 2021/085034 (06.05.2021 Gazette 2021/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.10.2019 JP 2019195671**

(71) Applicant: **FUJIFILM Corporation**
**Tokyo 106-8620 (JP)**

(72) Inventors:
• **MURAKAMI, Yuta**
**Ashigarakami-gun, Kanagawa 258-8577 (JP)**
• **OSAKI, Ryusuke**
**Ashigarakami-gun, Kanagawa 258-8577 (JP)**
• **ONOZAWA, Sho**
**Ashigarakami-gun, Kanagawa 258-8577 (JP)**
• **NAKAMURA, Sohichiro**
**Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

(54) **METHOD FOR SELECTING PLURIPOTENT STEM CELLS, METHOD FOR PREDICTING RESULT OF DIFFERENTIATION INDUCTION, AND METHOD FOR PRODUCING CELL PRODUCT**

(57)     There is provided a method including generating a phase contrast image of an aggregate of a pluripotent stem cell from a hologram in which the aggregate is captured; deriving, based on the phase contrast image, an index value indicating a complexity or simplicity of a contour line, the index value being determined according to a relationship between an area of the aggregate and a length of the contour line of the aggregate; and sorting the pluripotent stem cell based on the index value.

FIG. 10

$\beta = 0.71697\,\alpha + 3.28503$
$R^2 = 0.71796$
$R = 0.8473$

DIFFERENTIATION RATE (PREDICTED VALUE) [%]

DIFFERENTIATION RATE (MEASURED VALUE) [%]

EP 4 036 556 A1

**Description**

**BACKGROUND OF THE INVENTION**

1. Field of the Invention

The disclosed technology relates to a sorting method for a pluripotent stem cell, a prediction method for a differentiation induction result, and a production method for a cell.

2. Description of the Related Art

The following technology is known as a technology for determining a differentiation degree, which indicates a degree of differentiation of a pluripotent stem cell. For example, JP2015-146747A discloses that a cell is observed using a phase contrast microscope to obtain an optical path length N in the inside of the cell nucleus region and an optical path length C in the outside of the cell nucleus region, and a degree of differentiation of the cell is determined based on the ratio C/N, which is a ratio of the optical path length N to the optical path length.

Further, the following technology is known as a technology for determining the quality of induced pluripotent stem cells (iPS cells). For example, JP2018-000048A discloses an evaluation supporting method including a step of calculating a phase distribution of iPS cells from an image signal of a biological specimen of which an image is captured with a microscope that converts a phase distribution into an image intensity distribution, a step of extracting a region having a phase amount equal to or larger than a specific phase amount from the phase distribution, and a step of creating evaluation information serving as an indicator for evaluating the state of the iPS cells and presenting the evaluation information, using the region having a phase amount equal to or larger than a specific phase amount.

Furthermore, there is known a technology for determining whether pluripotent stem cells under being cultured maintain an undifferentiated state or are in a state deviated from the undifferentiated state. For example, WO2018/158901A discloses a cell analysis method characterized by executing a cell region extraction step of extracting a cell region in which cells are present in a phase image of a cell to be analyzed obtained from a hologram obtained with a holographic microscope; a background value acquisition step of calculating a background value based on phase values at a plurality of positions in a region other than the cell region in the phase image; an intracellular phase value acquisition step of obtaining a intracellular phase value based on phase values at a plurality of positions within a measurement target range between a contour line of a cell in the cell region and a virtual line inwardly spaced apart from the contour line by a predetermined distance; and a cell state determination step of determining whether the cell to be analyzed is in an undifferentiated state or a state deviated from the undifferentiated state, based on the difference between the phase value obtained in the intracellular phase value acquisition step and the background value.

**SUMMARY OF THE INVENTION**

Pluripotent stem cells such as an embryonic stem cell (an ES cell) and an iPS cell have an ability to differentiate into various kinds of cells, and thus they can be applied to drug discovery and regenerative medicine. For example, a cell product that is used in regenerative medicine is obtained by culturing and proliferating an iPS cell and carrying out a differentiation induction treatment for differentiating it into a target cell.

In the process of establishing an iPS cell, there is generally a step of separating a cell group into which reprogramming genes have been introduced into single cells and culturing each of the single cells thereby being cloned. Since obtained clones differ in degrees of in the change in gene expression profile and the change in DNA methylation pattern in association with reprogramming, the acquisition rate and production efficiency may vary significantly even in a case where, by using the same protocol, they are subsequently induced to differentiate into a specific cell such as a myocardial cell. At present, it is difficult to make the differences present between these clones uniform, and it is customary to select and culture a clone most suitable for production. In addition, even in a case where the same clone is used for production, the acquisition rate and productivity often differ for each production and batch due to the influences of the incubator's technology and protocol robustness, and thus it is not easy to analyze the factors thereof and take countermeasures.

Regarding the production period of a cell product, it takes several weeks to one month to obtain a specific cell such as a myocardial cell by carrying out a differentiation induction treatment on a pluripotent stem cell such as an iPS cell. Moreover, culture media that are used in cell culture and drugs that are used in the differentiation induction treatment are expensive. However, in a case where it is possible to sort a pluripotent stem cell that is expected to have a high acquisition rate or to predict a differentiation induction result, at a relatively early stage in the production process of the cell product, it is possible to take measures regarding pluripotent stem cells that cannot be expected to have a high acquisition rate, for example, discontinuing treatments or changing production conditions, whereby it is possible to increase the productivity of the cell product and reduce the production cost.

The disclosed technology has been made in consideration of the above-described points, and an object of the disclosed technology is to provide a sorting method for a pluripotent stem cell, which can contribute to the improvement of productivity of a cell product, a prediction method for a differentiation induction result, and a production method for a cell product.

A sorting method according to the disclosed technology comprises generating a phase contrast image of an aggregate of a pluripotent stem cell from a hologram in which the aggregate is captured; deriving, based on the phase contrast image, an index value indicating a complexity or simplicity of a contour line, the index value being determined according to a relationship between an area of the aggregate and a length of the contour line of the aggregate; and sorting the pluripotent stem cell based on the index value. The complexity of the contour line decreases as the contour of the aggregate becomes closer to a circle.

The index value may be a value that increases as the complexity of the contour line increases. In this case, a pluripotent stem cell contained in an aggregate having an index value smaller than a predetermined threshold value may be targeted for a differentiation induction treatment for differentiation into a specific cell. In addition, a clone containing an aggregate having an index value smaller than a predetermined threshold value, a production batch containing an aggregate having an index value smaller than a predetermined threshold value, a plate containing an aggregate having an index value smaller than a predetermined threshold value, or an aggregate having an index value smaller than a predetermined threshold value may be targeted for a differentiation induction treatment.

The index value may be a value that increases as the simplicity of the contour line increases. In this case, a pluripotent stem cell contained in an aggregate having an index value larger than a predetermined threshold value may be targeted for a differentiation induction treatment for differentiation into a specific cell. In addition, a clone containing an aggregate having an index value larger than a predetermined threshold value, a production batch containing an aggregate having an index value larger than a predetermined threshold value, a plate containing an aggregate having an index value larger than a predetermined threshold value, or an aggregate having an index value larger than a predetermined threshold value may be targeted for a differentiation induction treatment.

The threshold value may be determined based on a relationship between the index value derived in regard to the aggregate before carrying out the differentiation induction treatment and a differentiation rate indicating a proportion of the number of the specific cells with respect to all cells at a time after carrying out the differentiation induction treatment on the pluripotent stem cell.

A prediction method for a differentiation induction result according to the disclosed technology comprises generating a phase contrast image of an aggregate of a pluripotent stem cell from a hologram in which the aggregate is captured; deriving, based on the phase contrast image, an index value indicating a complexity or simplicity of a contour line, the index value being determined according to a relationship between an area of the aggregate and a length of the contour line of the aggregate; and deriving, based on the index value, a predicted value regarding the number of specific cells obtained by carrying out a differentiation induction treatment for differentiating the pluripotent stem cell into the specific cell.

For example, the predicted value may be a differentiation rate indicating a proportion of the number of the specific cells with respect to all cells at a time after carrying out the differentiation induction treatment on the pluripotent stem cell.

A function that shows a relationship between the index value and the differentiation rate may be used to derive the predicted value.

A production method for a cell product according to the disclosed technology comprises a culture step of culturing a pluripotent stem cell; a sorting step of sorting the pluripotent stem cell cultured in the culture step; and a differentiation induction step of carrying out a differentiation induction treatment for differentiating the pluripotent stem cell sorted in the sorting step into a specific cell. The sorting step includes generating a phase contrast image of an aggregate of a pluripotent stem cell from a hologram in which the aggregate is captured and deriving, based on the phase contrast image, an index value indicating a complexity or simplicity of a contour line, the index value being determined according to a relationship between an area of the aggregate and a length of the contour line of the aggregate.

The sorting step further includes sorting the pluripotent stem cell based on the index value.

In each of the methods according to the disclosed technology, the phase contrast image may be generated from the hologram of aggregates of the pluripotent stem cells in a state of being adhered on a base material, which is captured before carrying out the differentiation induction treatment.

In each of the methods according to the disclosed technology, in the phase contrast image, in a case where a threshold value is set, a first region having a phase contrast amount larger than the threshold value is regarded as a cell region in which cells are present, and a second region having a phase contrast amount smaller than the threshold value is regarded as a background, a total A of areas of the first region surrounded by a boundary line between the first region and the second region may be derived as an area of the aggregate, and a total P of lengths of the boundary lines may be derived as a length of the contour line of the aggregate.

For example, $\gamma$ given according to Expression (I) may be derived as the index value indicating the complexity of the contour line.

$$\gamma = P/2(\pi A)^{1/2} \quad \cdots (I)$$

In addition, ε given according to Expression (II) may be derived as the index value indicating the complexity of the contour line.

$$\varepsilon = P^2/4\pi A \quad \cdots (II)$$

In addition, ρ given according to Expression (III) may be derived as the index value indicating the simplicity of the contour line.

$$\rho = 2(\pi A)^{1/2}/P \quad \cdots (III)$$

Further, c given according to Expression (IV) may be derived as the index value indicating the simplicity of the contour line.

$$c = 4\pi A/P^2 \quad \cdots (IV)$$

Each of the methods according to the disclosed technology is the method according to any one of claims 2 to 14, in which the specific cell is a myocardial cell.
According to the disclosed technology, there is provided a sorting method for a pluripotent stem cell, which can contribute to the improvement of productivity of a cell product, a prediction method for a differentiation induction result, and a production method for a cell product.

## BRIEF DESCRIPTION OF THE DRAWINGS

Fig. 1 is a step flow chart showing one example of a production method for a cell product.
Fig. 2 is a flow chart showing one example of a processing flow in a sorting step according to an embodiment of the disclosed technology.
Fig. 3 is a view illustrating one example of an image capturing system according to the embodiment of the disclosed technology.
Fig. 4A is an image showing one example of a hologram of a plurality of spheres according to the embodiment of the disclosed technology.
Fig. 4B is an image showing one example of a Fourier transformed image of a plurality of spheres according to the embodiment of the disclosed technology.
Fig. 4C is an image showing one example of a phase contrast image of a plurality of spheres before unwrapping according to the embodiment of the disclosed technology.
Fig. 4D is an image showing one example of a phase contrast image of a plurality of spheres after unwrapping according to the embodiment of the disclosed technology.
Fig. 5 is a view illustrating a concept of a phase contrast image according to the embodiment of the disclosed technology.
In Fig. 6, a graph on the left is a graph illustrating one example of a relationship between the position in the plane direction and the phase contrast amount in a phase contrast image of spheres, and a graph on the right is a histogram of the phase contrast amount in the phase contrast image of the sphere.
Fig. 7 is a graph showing one example of a relationship between the focal position and the variation in the phase contrast amount in phase contrast images of spheres.
Fig. 8A is an image showing one example of a phase contrast image generated from a hologram of colonies adhered on a base material according to the embodiment of the disclosed technology.
Fig. 8B is an image showing one example of a phase contrast image subjected to binarization processing according to the embodiment of the disclosed technology.
Fig. 8C is an image showing one example of a contour line of a colony according to the embodiment of the disclosed

EP 4 036 556 A1

technology.

Fig. 9 is a graph showing one example of a relationship between an index value indicating the complexity of the contour line of a colony and a differentiation rate according to the embodiment of the disclosed technology.

Fig. 10 is a graph showing a relationship between a predicted value of a differentiation rate and a measured value thereof according to the disclosed technology.

Fig. 11A is a phase contrast microscopic image of a colony of iPS cells adhered on a base material according to Comparative Example.

Fig. 11B is an image showing a cell region according to Comparative Example and a region other than the cell region.

Fig. 12 is a graph showing one example of a relationship between an index value indicating the complexity of the contour line of a colony and a differentiation rate according to Comparative Example.

Fig. 13 is a graph showing a relationship between a predicted value of a differentiation rate and a measured value thereof according to Comparative Example.

**DESCRIPTION OF THE PREFERRED EMBODIMENTS**

Hereinafter, embodiments of the disclosed technology will be described with reference to the drawings. It is noted that in each of the drawings, substantially the same or equivalent constitution elements or parts are designated by the same reference numeral.

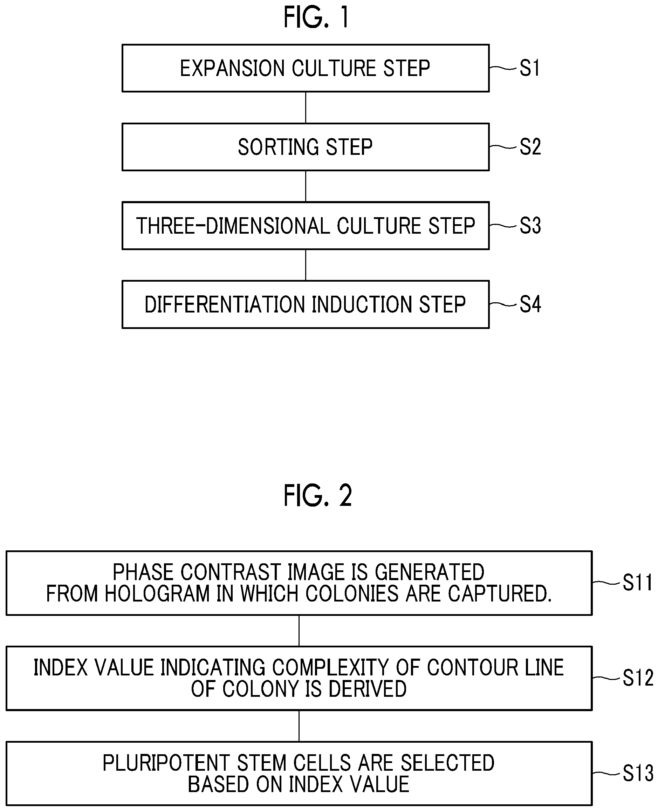

Fig. 1 is a step flow chart illustrating one example of a production method for a cell product according to the embodiment of the disclosed technology. The production method for a cell product according to the present embodiment is a production method for obtaining a specific cell that has deviated from the undifferentiated state, such as a myocardial cell, by differentiation induction of a pluripotent stem cell such as an iPS cell. The production method for a cell product according to the present embodiment includes an expansion culture step S1, a sorting step S2, a three-dimensional culture step S3, and a differentiation induction step S4.

In the expansion culture step S1, the expansion culture of proliferating a pluripotent stem cell is carried out. The expansion culture is carried out, for example, by two-dimensional culture in which a plurality of pluripotent stem cells are adhered on a base material. As the base material, it is possible to use, for example, a commercially available multi-well plate for cell culture. Pluripotent stem cells form an aggregate (hereinafter, referred to as a colony) on the base material. In the expansion culture step S1, culture medium exchange and subculture treatment are carried out a plurality of times until the desired number of pluripotent stem cells are obtained.

In the sorting step S2, a pluripotent stem cell is sorted. Specifically, a pluripotent stem cell to be targeted for a differentiation induction treatment that is carried out in the differentiation induction step S4 is specified. Details of the sorting method for a pluripotent stem cell will be described later.

In the three-dimensional culture step S3, the pluripotent stem cell is cultured to form a spherical aggregate (hereinafter, referred to as a sphere). During the transition from the two-dimensional culture to the three-dimensional culture, pluripotent stem cells adhered on the base material are detached from the base material using an enzyme agent such as trypsin. The detached pluripotent stem cells are subjected to, for example, spinner culture in a container containing a culture medium. The pluripotent stem cells form spherical spheres while being proliferated in a container. It is noted that in this step, it is also possible to apply stationary culture in which pluripotent stem cells are cultured in a stationary state in a culture medium. In a case of stationary culture, a plate subjected to a non-cell adhesive surface treatment may be used, or a material for preventing sphere sediment may be added to the culture medium. Examples of the sedimentation prevention material include heteropolysaccharides polymers such as Gellan Gum.

In the differentiation induction step S4, the pluripotent stem cells targeted for a differentiation induction treatment in the sorting step S2 are subjected to a differentiation induction treatment for differentiation into specific cells. The differentiation induction treatment is carried out at the timing when a predetermined period (for example, 2 days) has elapsed from the start of the three-dimensional culture step S2. For example, in a case where myocardial cells are obtained from pluripotent stem cells, a physiologically active substance that induces differentiation into mesoderm is added to the culture medium. Specific examples of the physiologically active substance include bFGF, Activin, BMP4. Then, a Wnt signal inhibitor that induces differentiation into myocardial cells is added to the culture medium. Specific examples of the Wnt signal inhibitor include XAV939.

Fig. 2 is a flow chart showing a processing flow that is carried out in the sorting step S2. In a step S11, a phase contrast image is generated from a hologram in which colonies adhered on the base material are captured. Holography is carried out, for example, on the colonies immediately before the transition to the three-dimensional culture step S3. Details of the holography and the phase contrast image will be described later.

In a step S12, an index value indicating the complexity of the contour line of the colony is derived based on the phase contrast image generated in the step S11. The index value indicating the complexity of the contour line of the colony is a numerical value determined according to the relationship between the area of the colony and the length of the contour line of the colony, and it has a larger value as the complexity of the contour line of the colony increases.

5

That is, the complexity of the contour line of the colony decreases as the colony contour line of the colony becomes closer to a circle. The details of the index value indicating the complexity of the contour line of the colony will be described later.

In a step S13, pluripotent stem cells are sorted based on the index value indicating the complexity of the contour line of the colony derived in step the S12. Specifically, in a case where the index value indicating the complexity of the contour line of the colony is smaller than a predetermined threshold value (that is, in a case where the contour line of the colony is relatively smooth), pluripotent stem cells contained in this colony are targeted for a differentiation induction treatment. On the other hand, in a case where the index value indicating the complexity of the contour line of the colony is larger than the above-described threshold value (that is, in a case where the contour line of the colony is relatively intricate), pluripotent stem cells contained in this colony are excluded from targets for the differentiation induction treatment. It is noted that the pluripotent stem cells excluded from the targets for the differentiation induction treatment may also be excluded from targets for the three-dimensional culture.

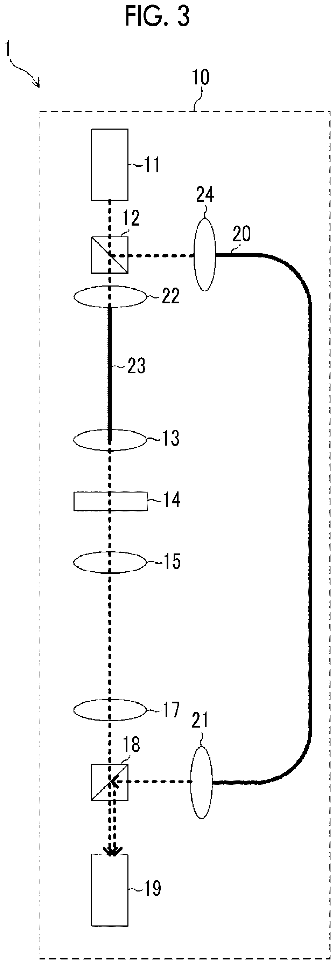

Fig. 3 is a view illustrating one example of a constitution of an image capturing system 1 that is used for sorting a pluripotent stem cell in the sorting step S2. The image capturing system 1 is constituted by including a hologram optical system 10 for acquiring a hologram of aggregates (colonies or spheres) of pluripotent stem cells using a known digital holography technology,

The digital holography technology is a technology that restores wavefronts of light waves from an object by capturing, with an image sensor, an image generated due to the interference between the object light that has penetrated through or reflected from the object and the reference light that is coherent to the object light and subjecting an image obtained by the image capturing to a numerical calculation based on light propagation. According to the digital holography technology, it is possible to quantify the phase distribution of an object and acquire three-dimensional information of the object without mechanically moving the focal position.

A hologram optical system 10 includes a laser beam source 11, beam splitters 12 and 18, collimating lens 13, 21, 22, and 24, an objective lens 15, an imaging lens 17, and a complementary metal oxide semiconductor (CMOS) camera 19. An aggregate as a sample 14 set on a sample stage is arranged between the collimating lens 13 and the objective lens 15.

As the laser beam source 11, it is possible to use, for example, a HeNe laser having a wavelength of 632.8 nm. The laser beams emitted from the laser beam source 11 are split into two laser beams by the beam splitter 12. One of the two laser beams serves as object light and the other thereof serves as reference light. The object light is incident on an optical fiber 23 by the collimating lens 22 and guided to the front of the collimating lens 13 by the optical fiber 23. The object light is made to be parallel light by the collimating lens 13 and then emitted on the aggregate which is the sample 14 set on the sample stage. As the optical fiber 23, it is possible to use, for example, an optical fiber having NA = 0.11. As the collimating lens 13, it is possible to use, for example, a collimating lens having f = 10 mm (NA = 0.4). The diameter of the laser beam that is emitted to the aggregate is, for example, 2.2 mm. The image generated due to the object light penetrated through the aggregate is magnified by the objective lens 15. The object light penetrated through the objective lens 15 is made to be parallel light again by the imaging lens 17 and then an image is formed on the imaging surface of the CMOS camera 19 through the beam splitter 18. As the objective lens 15, it is possible to use, for example, an object lens having NA = 0.20 and f = 50 mm. As the imaging lens 17, it is possible to use, for example, an object lens having f = 200 mm and an opening diameter of 36 nm, and an image is formed on the imaging surface of the CMOS camera at an image magnification of 4 times. On the other hand, the reference light is incident on an optical fiber 20 by the collimating lens 24 and guided to the front of the collimating lens 21 by the optical fiber 20. The reference light emitted from the optical fiber 20 is made to be parallel light by the collimating lens 21 and is incident on the imaging surface of the CMOS camera 19 through the beam splitter 18. As the collimating lens 21, it is possible to use, for example, a collimating lens having f = 100 mm (NA = 0.25). The diameter of the laser beam that is emitted from the collimating lens 21 is, for example, 22 mm. A hologram generated due to the interference between the object light and the reference light is recorded by the CMOS camera 19. As the CMOS camera 19, it is possible to use, for example, a monochrome image sensor having a resolution of 2,448 $\times$ 2,048 and a sensor size of 3.45 $\mu$m $\times$ 3.45 $\mu$m. Image capturing may be carried out by tilting the beam splitter 18 so that the reference light is tilted by about 3° with respect to the object light, so that an off-axial optical system in which optical axis directions of the object light and the reference light, incident on the imaging surface of the CMOS camera 19, are different from each other.

According to the image capturing system 1 according to the present embodiment, it is possible to acquire a phase contrast image of a colony without destroying the aggregate and without damaging cells that constitute the colony. It is noted that the constitution of the image capturing system 1 described above is merely one example and thus is not limited to the constitution described above. For carrying out the sorting method according to the present embodiment, it is possible to use any image capturing system capable of acquiring a hologram by using digital hologram technology.

Hereinafter, a description will be made for one example of a method of acquiring a phase contrast image of an aggregate from a hologram of aggregates of pluripotent stem cells acquired by using the image capturing system 1.

First, a hologram of aggregates of iPS cells, acquired by the image capturing system 1 and exemplified in Fig.

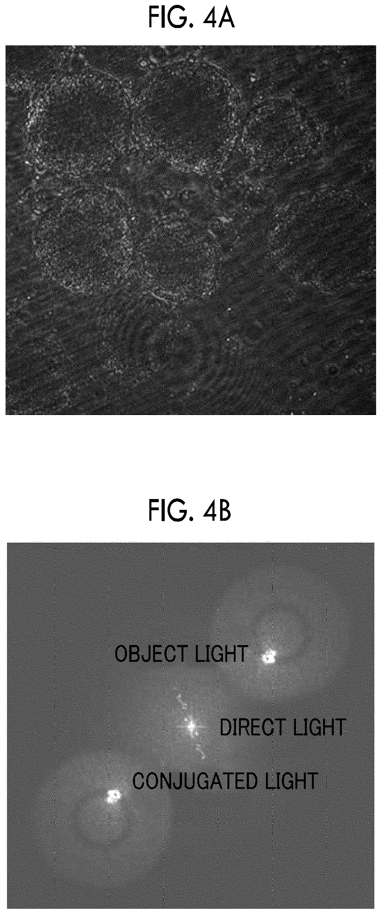

4A, is trimmed to a size of, for example, 2,048 × 2,048 and then subjected to a two-dimensional Fourier transform. Fig. 4B is one example of a Fourier transformed image of the aggregates obtained by this processing. Fig. 4B shows an image based on direct light, object light, and conjugated light.

Next, the position of the object light is specified by specifying the amount of deviation of the object light with respect to the direct light in the Fourier transformed image, and the complex amplitude component of only the object light is extracted by, for example, the frequency filtering process using a mask having a circular opening of a radius of 250 pixels.

Next, for example, the angular spectral method is applied to restore an image showing the phase of the aggregate at any spatial position. Specifically, the angular spectrum U ($f_x$, $f_y$; 0) of the Fourier transformed image of the wavefront u (x, y; 0) captured on the imaging surface of the CMOS camera 19 is determined. Next, as shown in Expression (1) below, a transfer function H ($f_x$, $f_y$; z) is multiplied by the angular spectrum U ($f_x$, $f_y$; 0) to reproduce the wavefront at any position z in the optical axis direction (the z direction) of the image capturing system 1. Here, the transfer function H ($f_x$, $f_y$; z) is a frequency response function (a Fourier transform of the impulse response function (the Green's function)).

$$U\left(f_x, f_y; z\right) = U\left(f_x, f_y; 0\right) H\left(f_x, f_y; z\right), \quad H = e^{\frac{2\pi}{\lambda}\sqrt{1-(\lambda f_x)^2-(\lambda f_y)^2}} \quad \cdots (1)$$

Next, as shown in Expression (2), the wavefront U ($f_x$, $f_y$; z) at the position z in the optical axis direction (the z direction) of the image capturing system 1 is subjected to the inverse Fourier transform, whereby a solution u (x, y; z) at the position z is derived.

$$\begin{aligned} u(x, y; z) &= F^{-1}\left[U\left(f_x, f_y; z\right)\right] \\ &= F^{-1}\left[U\left(f_x, f_y; 0\right) H\left(f_x, f_y; z\right)\right] \\ &= F^{-1}\left[F\left[u(x, y; 0)\right] H\left(f_x, f_y; z\right)\right] \end{aligned} \quad \cdots (2)$$

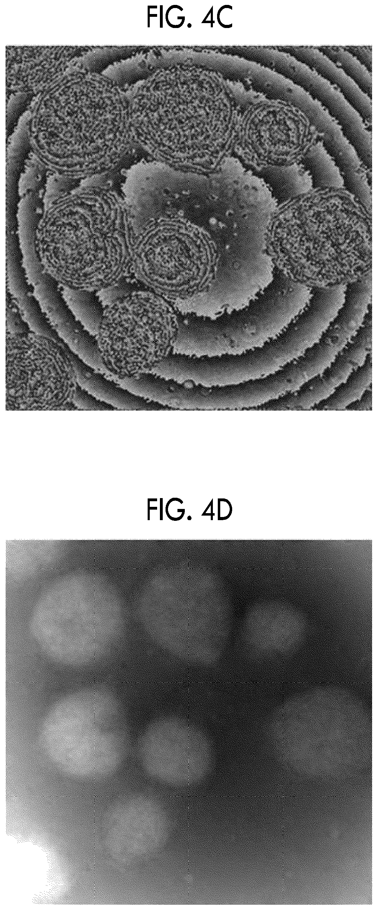

Next, as shown in Expression (3), a phase contrast image is generated by deriving the phase φ for u (x, y; z). Fig. 4C is one example of a phase contrast image of the aggregates before unwrapping, obtained by each processing described above.

$$\phi = \arctan\left(\frac{\mathrm{Im}(u)}{\mathrm{Re}(u)}\right) \quad \cdots (3)$$

The phase of the aggregate before unwrapping shown in Fig. 4C is convoluted to a value of 0 to $2\pi$. Here, in a case where a part of $2\pi$ or more is joined by applying a phase connection (unwrapping) method, for example, the unweighted least squares method or the Flynn's algorithm, it is possible to obtain a final phase contrast image of the aggregates as shown in Fig. 4D. It is noted that a large number of unwrapping methods have been proposed, and a suitable method that does not cause phase mismatch may be appropriately selected.

In a case where each processing described above is carried out, it is possible to generate a phase contrast image at each of the positions z different from each other in the optical axis direction (the z direction) of the image capturing system 1.

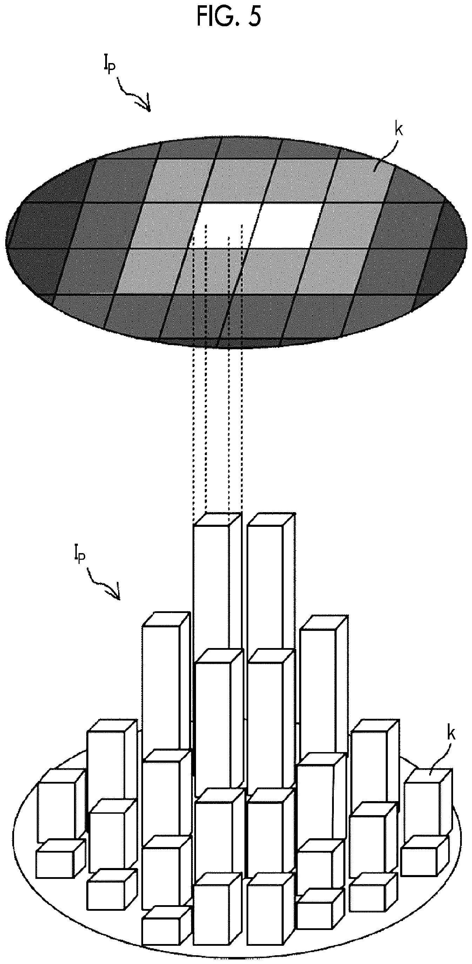

Hereinafter, the phase contrast image will be described. Fig. 5 is a view illustrating a concept of a phase contrast image Ip. The lower part of Fig. 5 is a view in which a phase contrast amount in each pixel k of the phase contrast image Ip is three-dimensionally displayed. The upper part of Fig. 5 is a view in which the phase contrast amount in each pixel k of the phase contrast image Ip is illustrated on a plane in gray scale.

Here, in the same focal plane of the phase contrast image Ip, in a case where the phase of the background (the region where aggregates are not present) present is denoted by $P_B$, and the phase of the region where aggregates are present is denoted by Ps, a phase contrast amount P in the phase contrast image $I_P$ is expressed by Expression (4). It is noted that the term "phase" in the present specification is the phase of the electric field amplitude in a case where light is regarded as an electromagnetic wave and is used in a more general meaning.

$$P = P_S - P_B \quad \cdots \ (4)$$

Further, a phase contrast amount $P_k$ in each pixel k of the phase contrast image Ip can be expressed by Expression (5). Here, $n_k$ is the refractive index of the aggregate at the portion corresponding to each pixel k of the phase contrast image $I_P$, $d_k$ is the thickness of the aggregate at the portion corresponding to each pixel k of the phase contrast image $I_P$, and $\lambda$ is the wavelength of the object light in the hologram optical system 10.

$$P_k = 2\pi \frac{n_k \cdot d_k}{\lambda} \quad \cdots \ (5)$$

The phase contrast image of the aggregate is an image showing the optical path length distribution of the object light penetrated through the aggregate. Since the optical path length in the aggregate corresponds to the product of the refractive index of the aggregate and the thickness of the aggregate, the phase contrast image of the aggregate contains information on the refractive index and the thickness (the shape) of the aggregate, as also shown in Expression (5).

From the phase contrast image that is out of focus with respect to the aggregate, it is not possible to obtain accurate information that matches the actual condition of the aggregate due to the influence of the spread caused by diffraction. As a result, it is preferable to focus on the aggregate in a case of acquiring a phase contrast image from the hologram acquired by the CMOS camera 19. Here, "to focus on the aggregate" means to obtain a phase contrast image that is sliced near the center of the spherical aggregate. In a case where the state of the aggregate is determined using a phase contrast image in which the aggregate is in focus, it is possible to obtain a more accurate determination result.

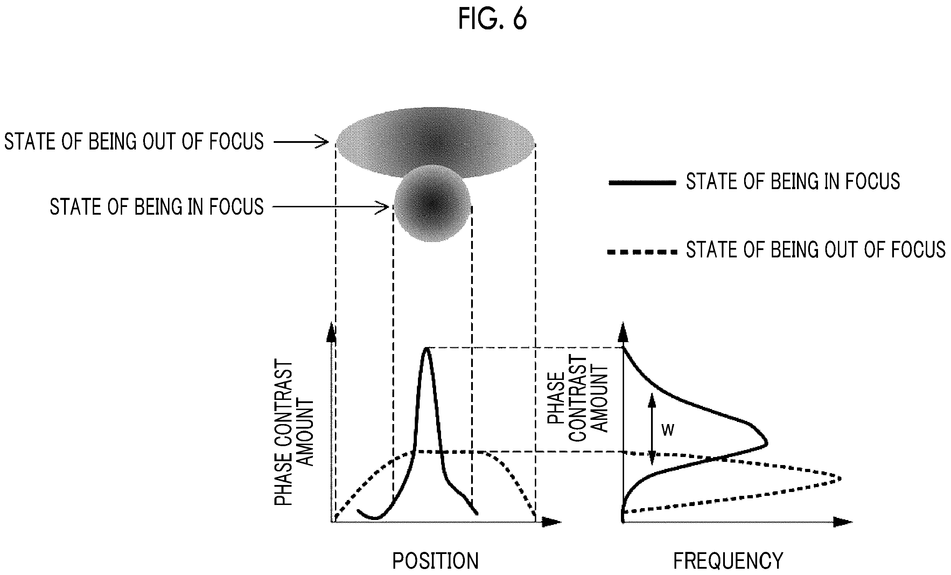

The graph on the left of Fig. 6 is a graph showing one example of the relationship between the position in the plane direction and the phase contrast amount of the aggregate in the phase contrast image, where the solid line corresponds to a state where the aggregate is in focus and the dotted line corresponds to a state where the aggregate is out of focus. In a case where the aggregate is in focus, a steep peak appears at a specific position in the phase contrast image. On the other hand, in a case where the aggregate is out of focus, a peak is low and gentle as compared with the case of being in focus.

The graph on the right of Fig.6 is a histogram of the phase contrast amount in the phase contrast image of the aggregate, where the solid line corresponds to a state where the aggregate is in focus and the dotted line corresponds to a state where the aggregate is out of focus. In a case where the aggregate is in focus, the curve width w (the variation in the phase contrast amount) is relatively large, and in a case where the aggregate is out of focus, the curve width w (the variation in the phase contrast amount) is relatively small.

As a result, focusing can be achieved by acquiring the phase contrast image of the aggregate for each of the focal positions (slice positions) different from each other, determining the curve width w (the variation in the phase contrast amount) in the histogram of the phase contrast amount for each acquired phase contrast image, and extracting a phase contrast image having the maximum width w among the determined widths w as the phase contrast image in which the aggregate is in focus.

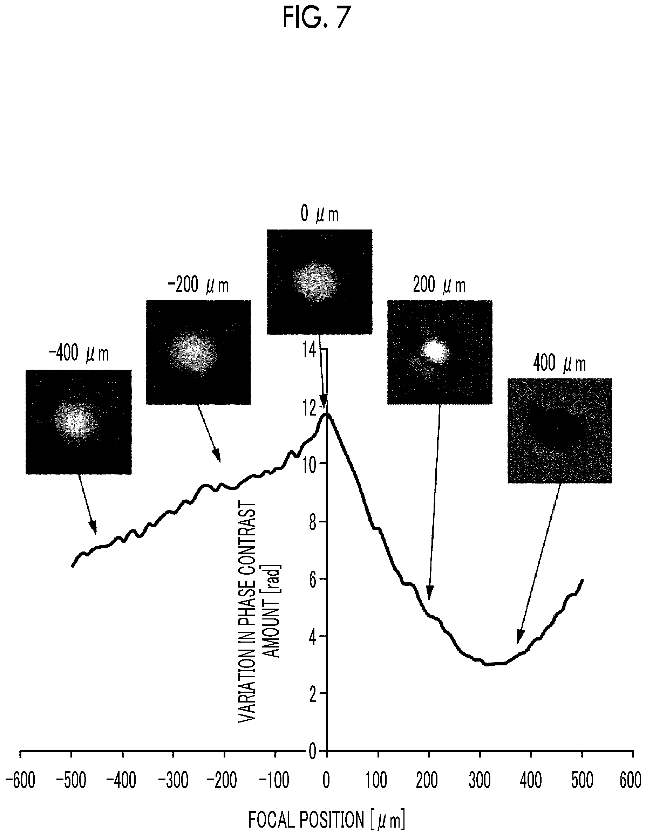

Fig. 7 is a graph showing one example of a relationship between the focal position (the slice position) and the variation in the phase contrast amount in phase contrast images of aggregates. In Fig. 7, phase contrast images of aggregates corresponding to focal positions of -400 $\mu$m, -200 $\mu$m, 0 $\mu$m, +200 $\mu$m, and +400 $\mu$m are exemplified together with the graph. In Fig. 7, the focal position where the variation in the phase contrast amount has the maximum value is set to 0 $\mu$m. According to the above-described autofocus processing, a phase contrast image corresponding to a focal position of 0 $\mu$m where the variation in the phase contrast amount has the maximum value is extracted as the in-focus phase contrast image. In the phase contrast image corresponding to a focal position of 0 $\mu$m where the variation in the phase contrast amount has the maximum value, the contour of the aggregate becomes clearest.

Hereinafter, a description will be made for one example of a method of deriving an index value indicating the complexity of the contour line of the colony of pluripotent stem cells based on the phase contrast image of this colony.

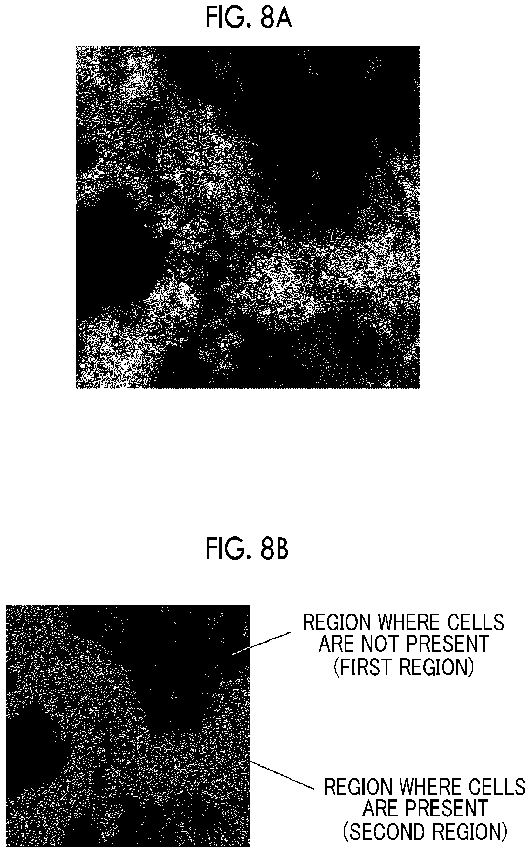

Fig. 8A is an image showing one example of a phase contrast image generated from a hologram of colonies adhered on a base material. The phase contrast image shown in Fig. 8A is subjected to binarization processing. For example, "1" is assigned to a pixel having a phase contrast amount larger than a predetermined threshold value, and "0" is assigned to a pixel having a phase contrast amount smaller than the threshold value. In the phase contrast image, the first region in which the phase contrast amount is larger than the threshold value is a region in which cells are present, and the second region in which the phase contrast amount is smaller than the threshold value is a region (a background) in which cells are not present. Fig. 8B is an image showing one example of a phase contrast image subjected to binarization processing.

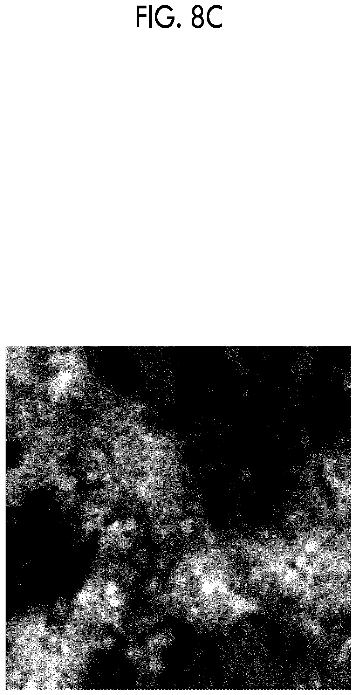

Next, a contour line of the colony is extracted from the binarized phase contrast image. Specifically, a boundary line between the first region (the region in which cells are present) in which the phase contrast amount is larger than the threshold value and the second region (the region in which cells are not present) in which the phase contrast amount is smaller than the threshold value is extracted as the contour line of the colony. Fig. 8C is an image showing one example of an extracted contour line.

Next, a total A of areas of the first region surrounded by a boundary line between the first region and the second region is derived as the area of the colony. Next, a total P of lengths of the boundary lines between the first region and the second region is derived as the length of the contour line of the colony.

Next, $\gamma$ given according to Expression (6) is derived as the index value indicating the complexity of the contour line of the colony. The $\gamma$ given according to Expression (6) becomes larger as the contour line of the colony becomes more complicated. The complexity of the contour line of a colony reflects the randomness of the phase contrast amount in the outer peripheral part of the colony.

$$\gamma = \frac{P}{2\sqrt{\pi A}} \quad \cdots (6)$$

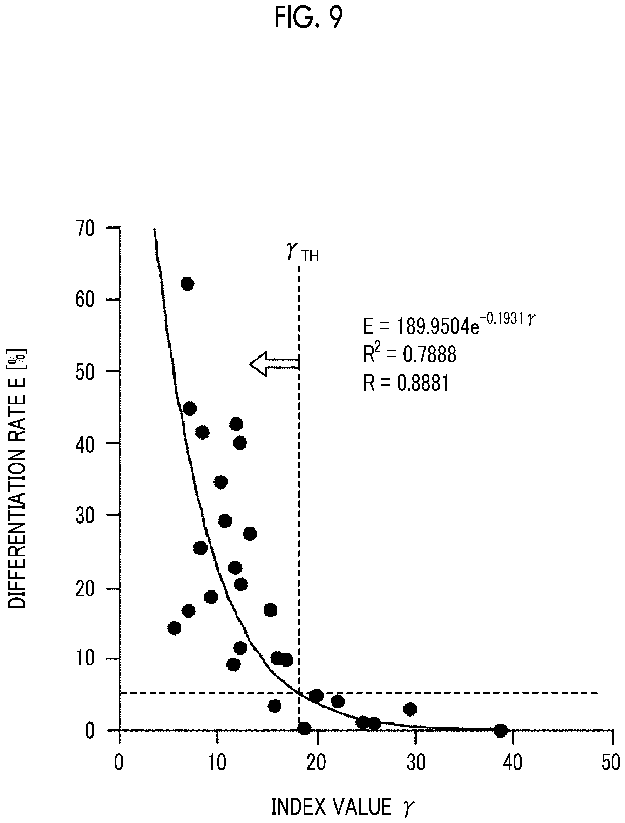

Fig. 9 is a graph showing one example of the relationship between the index value $\gamma$ indicating the complexity of the contour line of a colony of iPS cells and the differentiation rate E of the myocardial cell obtained by the differentiation induction treatment on the iPS cells contained in this colony. The index value $\gamma$ is a value derived from the phase contrast image generated from the hologram acquired in regard to the colony in a state of being adhered on the base material immediately before the transition to the three-dimensional culture step S3. Each plot shown in Fig. 9 corresponds to each of a plurality of production lots in the production process of the cell product, and the index value $\gamma$ indicated by each plot is the average value of the index values $\gamma$ acquired in regard to a plurality of colonies in the production lot.

The differentiation rate E is expressed by Expression (7). In Expression (7), $N_a$ is the number of all cells including pluripotent stem cells and myocardial cells in the production lot obtained at a time after carrying out the differentiation induction treatment (12 days after the start of the differentiation induction step S3 in the present embodiment), and $N_c$ is the number of myocardial cells in the production lot at the same time as the time when acquiring $N_i$. The number of myocardial cells $N_c$ can be acquired by measuring the number of cTnT positive cells.

$$E = \frac{N_c}{N_a} \times 100 \quad \cdots (7)$$

As shown in Fig. 9, the larger the index value $\gamma$ indicating the complexity of the contour line of the colony, the lower the differentiation rate E. That is, Fig. 9 shows that pluripotent stem cells having a high differentiation rate E can be sorted with the index value $\gamma$ indicating the complexity of the contour line of the colony.

The sorting method for a pluripotent stem cell according to the embodiment of the disclosed technology, illustrated in Fig. 2, is a sorting method based on the finding that the differentiation rate E is reflected in the index value $\gamma$ indicating the complexity of the contour line of the colony, as shown in Fig. 9, and it includes sorting the pluripotent stem cells based on the index value (see a step S13 in Fig. 2). Specifically, it includes targeting pluripotent stem cells contained in a colony in which the index value $\gamma$ indicating the complexity of the contour line of the colony is smaller than the predetermined threshold value $\gamma_{TH}$, for a differentiation induction treatment; and excluding pluripotent stem cells contained in a colony in which the index value $\gamma$ indicating the complexity of the contour line of the colony is larger than the above-described threshold value $\gamma_{TH}$ from targets for the differentiation induction treatment. It is noted that the pluripotent stem cells excluded from the targets for the differentiation induction treatment may also be excluded from targets for the three-dimensional culture.

The sorting method according to the embodiment of the disclosed technology includes determining the threshold value $\gamma_{TH}$ for the sorting in regard to the index value $\gamma$, based on the relationship between the index value $\gamma$ indicating the complexity of the contour line of the colony, acquired in regard to the colony before carrying out the differentiation induction treatment and the differentiation rate E in a case where the pluripotent stem cells contained in this colony have been subjected to the differentiation induction treatment, as exemplified in Fig. 9. For example, in the example shown in Fig. 9, in a case where the differentiation rate E is to be 5% or more, pluripotent stem cells contained in the colony in which the index value $\gamma$ indicating the complexity of the contour line of the colony is 19 or less are targeted for a differentiation induction treatment.

The sorting of pluripotent stem cells can be carried out on a colony basis or on a production lot basis. In a case where the sorting of pluripotent stem cells is carried out on a production lot basis, for example, the index value $\gamma$ is acquired in regard to a part or all of a plurality of colonies in the production lot, and then all pluripotent stem cells contained in this production lot may be targeted for a differentiation induction treatment in a case where the intra-lot average value of the index value $\gamma$ is smaller than the threshold value $\gamma_{TH}$.

According to the sorting method according to the embodiment of the disclosed technology, since pluripotent stem cells are sorted based on the index value indicating the complexity of the contour line of the colony, it is possible to specify a pluripotent stem cell that can be expected to have a desired differentiation rate at a relatively early stage in the production process of the cell product. As a result, it is possible to take measures regarding pluripotent stem cells that cannot be expected to have a desired differentiation rate, for example, discontinuing treatments or changing production conditions, whereby it is possible to increase the productivity of the cell product and reduce the production cost.

Here, the relationship between the index value $\gamma$ indicating the complexity of the contour line of the colony and the differentiation rate E can be mathematically expressed using a known function fitting method. For example, Expression (8) can be derived as a function that shows a relationship between the index value $\gamma$ indicating the complexity of the contour line of the colony and the differentiation rate E, shown in Fig. 9.

$$E = 189.9504e^{-0.1931\gamma} \quad \cdots (8)$$

Expression (8) shows that the differentiation rate E can be predicted from the index value $\gamma$ indicating the complexity of the contour line of the colony obtained in the middle stage in the production process of the cell product. The coefficient of determination $R^2$ in the function (the regression model) represented by Expression (8) is 0.7888, and the correlation coefficient R is 0.8881. The coefficient of determination $R^2$ is an indicator indicating how much the predicted value according to the regression model deviates from the measured value, and it takes a value of 0 or more and 1 or less. It is noted that the closer the coefficient of determination is to 1, the smaller the deviation between the predicted value and the measured value according to the regression model is. The correlation coefficient R is the square root of the coefficient of determination $R^2$.

The prediction method for a differentiation induction result according to the embodiment of the disclosed technology is a prediction method that utilizes the relationship between the index value $\gamma$ indicating the complexity of the contour line of the colony and the differentiation rate E, and it includes, based on the index value $\gamma$ indicating the complexity of the contour line of the colony, deriving a predicted value regarding the number of myocardial cells obtained by carrying out a differentiation induction treatment on a plurality of pluripotent stem cells contained in this colony. Specifically, it includes substituting the index value $\gamma$ indicating the complexity of the contour line of the colony in a function that shows a relationship between the index value $\gamma$ indicating the complexity of the contour line of the colony and the differentiation rate E, the function being exemplified in Expression (8), thereby deriving a predicted value of the differentiation rate E in a case where pluripotent stem cells contained in this colony have been subjected to the differentiation induction treatment.

A function that shows a relationship between the index value $\gamma$ indicating the complexity of the contour line of the colony and the differentiation rate E is specified in this manner, and thus based on the index value $\gamma$ indicating the complexity of the contour line of the colony, it is possible to derive a predicted value of the differentiation rate E in a case where pluripotent stem cells contained in this colony have been subjected to the differentiation induction treatment, that is, it is possible to predict the differentiation induction result. It is noted that it is also possible to derive a predicted value other than the differentiation rate E as the predicted value regarding the number of myocardial cells, by using the function that shows a relationship between the index value $\gamma$ indicating the complexity of the contour line of the colony and the differentiation rate E. For example, in a case where $N_a$ (the total cell number) in Expression (7) has been measured, it is also possible to derive a predicted value of the number of myocardial cells $N_c$ (= $N_a \times E$) to be produced, by using the function that shows a relationship between the index value $\gamma$ indicating the complexity of the contour line of the colony and the differentiation rate E.

As described above, according to the prediction method for the differentiation induction result according to the embodiment of the disclosed technology, a predicted value regarding the number of myocardial cells obtained by carrying out a differentiation induction treatment on pluripotent stem cells contained in the colony is derived based on the index value $\gamma$ indicating the complexity of the contour line of this colony. As a result, it is possible to take measures, for example, regarding pluripotent stem cells in which the predicted value regarding the number of myocardial cells is less than the required level, for example, discontinuing treatments or changing production conditions, whereby it is possible to increase the productivity of the cell product and reduce the production cost.

In the above description, a case where pluripotent stem cells are differentiated into myocardial cells has been exemplified; however, the disclosed technology is not limited to this aspect. It is conceived that the relationship between the index value $\gamma$ indicating the complexity of the contour line of the colony and the differentiation rate E, exemplified in Fig. 9, is also established in a case where pluripotent stem cells are differentiated into cells other than myocardial cells. Accordingly, it is conceived that the disclosed technology can be applied even in a case where pluripotent stem cells are differentiated into cells other than myocardial cells. Examples of the cell other than the myocardial cell include entodermal cells such as digestive system cell (a hepatocyte, cholangiocyte, a pancreatic endocrine cell, an acinar cell, a ductal cell, an absorptive cell, a goblet cell, a Paneth cell, an intestinal endocrine cells, and the like), and cells of tissues such as lung and thyroid, examples of the mesenchymal cell include blood cells and lymphoid cells (a hematopoietic stem cell, an erythrocyte, a platelet, a macrophage, a granulocyte, a helper T cell, a killer T cell, a B lymphocyte, and the like), vascular system cells (a vascular endothelial cell and the like), an osteoblast, a bone cell, a cartilage cell, a tendon cell, an adipocyte, a skeletal muscle cell, and a smooth muscle cell, and examples of the ectodermal cell include a neural cell, sensory organ (crystalline lens, retina, inner ear, and the like) cells, a skin epidermal cell, a hair follicle.

In addition, in the above description, a case of deriving an index value indicating the complexity of the contour line of an aggregate (a colony) of pluripotent stem cells, in a state of being adhered on the base material, has been exemplified; however, the disclosed technology is not limited to this aspect. It is presumed that the differentiation rate E is reflected not only in the complexity of the contour line of the colony but also in the complexity of the contour line of a spherical aggregate (a sphere) formed in the three-dimensional culture step S3. Accordingly, the aggregates (the spheres) of spherical pluripotent stem cells, formed in the three-dimensional culture step S3, may be subjected to holography, a phase contrast image of the spheres may be generated from the hologram, and then an index value indicating the complexity of the contour line of the sphere may be derived from this phase contrast image.

In addition, in the above description, a case where $\gamma$ given according to Expression (6) has been exemplified as the index value indicating the complexity of the contour line of the colony; however, the disclosed technology is not limited to this aspect. For example, it is also possible to apply $\varepsilon$ given according to Expression (9) as the index value indicating the complexity of the contour line of the colony. It is noted that $\varepsilon$ corresponds to the reciprocal of the circularity.

$$\varepsilon = \frac{P^2}{4\pi A} \quad \cdot \cdot \cdot (9)$$

Further, in the above description, a case where the sorting of pluripotent stem cells and the prediction of the differentiation induction result are carried out based on the index value indicating the complexity of the contour line of the colony has been exemplified; however, the disclosed technology is not limited to this aspect. For example, it is also possible to sort pluripotent stem cells and predict the differentiation induction result based on the index value indicating the simplicity (the smoothness) of the contour line of a colony. The index value indicating the simplicity of the contour line of a colony becomes larger as the simplicity of the contour line of the colony increases.

As an index value indicating the simplicity of the contour line of a colony, for example, it is also possible to apply $\rho$ given according to Expression (10) or c given according to Expression (11). It is noted that the $\rho$ given according to Expression (10) corresponds to the reciprocal of the $\gamma$ given according to Expression (6), and the c given according to Expression (11) corresponds to the reciprocal of the $\varepsilon$ given according to Expression (9) and corresponds to circularity.

$$\rho = \frac{2\sqrt{\pi A}}{P} \quad \cdot \cdot \cdot (10)$$

$$c = \frac{4\pi A}{P^2} \quad \cdots (11)$$

The larger the index value indicating the simplicity of the contour line of a colony, the higher the differentiation rate E. Accordingly, in a case of sorting pluripotent stem cells based on the index value indicating the simplicity of the contour line of a colony, pluripotent stem cells contained in the colony in which the index value is larger than a predetermined threshold value are targeted for a differentiation induction treatment.

Further, the index value indicating the complexity or simplicity of the contour line of a colony is not limited to those exemplified above, and various values that are determined depending on the relationship between the total A of areas of the colonies and the total P of lengths of the contour lines can be applied. The index value indicating the complexity or simplicity of the contour line of a colony is preferably, for example, a dimensionless value obtained by taking a ratio between A and P. It is also possible to take a ratio between A and P and apply a value obtained by normalizing the ratio with the size of the colony.

[Example]

Hereinafter, Examples regarding the derivation of a graph showing a relationship between the index value $\gamma$ indicating the complexity of the contour line of an iPS cell colony and the differentiation rate E, shown in Fig. 9, will be disclosed. It is noted that the disclosed technology is not limited to Examples below.

IPS cell clones A to Z and a to c were prepared from peripheral blood mononuclear cells derived from different donors according to the method described in JP5984217B. MTeSR1 (Stem Cell Technologies) and Matrigel (Corning Inc.) were used for culturing iPS cells, and cells were recovered and subcultured by treatment with a 0.5 mM EDTA solution (Thermo Fisher Scientific, Inc.) for 7 minutes. Under the above conditions, iPS cells were subjected to expansion culture up to a scale of two T225 flasks. Under the above conditions, the iPS cells were proliferated until the confluency reached about 80%.

Apart of cells of each of the 29 clones were sampled, a hologram of an iPS cell colony was acquired using an image capturing system 1, and a phase contrast image was generated in regard to the obtained hologram of the colony.

The constitution of the image capturing system 1 used is as follows. As the laser beam source 11, a HeNe laser having a wavelength of 632.8 nm and an output of 5 mW was used. As the optical fiber 23, an optical fiber having NA = 0.11 was used. As the collimating lens 13, a collimating lens having f = 10 mm (NA = 0.4) was used. The diameter of the laser beam that is emitted to the aggregate was set to 2.2 mm. As the objective lens 15, an objective lens having NA = 0.20 and f = 50 mm was used. As the imaging lens 17, an imaging lens having f = 200 mm and an opening diameter of 36 mm was used, and an image was formed on the imaging surface of the CMOS camera at an image magnification of 4 times. As the collimating lens 21, a collimating lens having f = 100 mm (NA = 0.25) was used. As the CMOS camera 19, a monochrome image sensor having a resolution of 2,448 $\times$ 2,048 and a sensor size of 3.45 $\mu$m $\times$ 3.45 $\mu$m was used. The image capturing was carried out by tilting the beam splitter 18 so that the reference light is tilted by about 3° with respect to the object light, so that an off-axial optical system in which optical axis directions of the object light and the reference light, incident on the imaging surface of the CMOS camera 19, were different from each other.

After trimming the image acquired by the image capturing system 1 to a size of 2,048 $\times$ 2,048, a two-dimensional Fourier transform was carried out, and the complex amplitude component of only the object light was extracted by the frequency filtering process using a mask having a circular opening of a radius of 250 pixels. The angular spectral propagation method was used to restore the phase contrast image. The unweighted least squares method was used for phase unwrapping. The phase contrast image was subjected to binarization processing, and the contour line of the colony was extracted. The total A of areas of the regions surrounded by the contour line was derived as the area of the colony, and the total P of lengths of the contour lines was derived as the length of the contour line of the colony. Regarding each of the 29 clones, the index value $\gamma$ indicating the complexity of the contour line of the colony was derived using Expression (6).

The cells proliferated by expansion culture were recovered by being detached to be single cells with TrypLE Select (Thermo Fisher Scientific, Inc.), and the cell concentration was adjusted to 3.0 $\times$ 10$^6$ cells/ml in the mTeSR1 to which 1 $\mu$M H1152 (FUJIFILM Wako Pure Chemical Corporation), 25 $\mu$g/ml Gentamicin (Thermo Fisher Scientific, Inc.), and 100 ng/ml bFGF (FUJIFILM Wako Pure Chemical Corporation) had been added in terms of final concentration. 15 ml of the cell suspension was added to a single-use bioreactor (ABLE Corporation) having a capacity of 30 ml and subjected to spinner culture at a rotation speed of 40 rpm. After 2 to 4 hours from the start of the culture, the cell suspension was adjusted to a final liquid volume of 30 ml in the same culture medium and continuously subjected to the spinner culture as it was.

1 day after the start of the spinner culture, the culture medium was exchanged to a culture medium consisting of 1 $\mu$M H1152, 25 $\mu$g/ml Gentamicin, 100 ng/ml bFGF, 24 ng/ml Activin, 5% fetal bovine serum (GE Healthcare), ×0.5 mTeSR1, and ×0.5 DMEM Low-glucose (Thermo Fisher Scientific, Inc.) in terms of final concentration, and then spinner culture was continued.

Two days after the start of the spinner culture, a part of the culture solution was sampled to measure the number of cells, the culture solution was adjusted to 1.0 × 10$^6$ cells/ml in a culture medium consisting of 25 $\mu$g/ml Gentamaicin, 100 ng/ml bFGF, 24 ng/ml Activin, 40 ng/ml BMP4, 10% fetal bovine serum, and DMEM low-glucose in terms of final concentration, and then differentiation culture was started. The spinner culture was continued even during the differentiation culture. From 3 days to 7 days after the start of the spinner culture, the culture medium was changed daily with the same culture medium, and then the spinner culture was continued.

Eight days after the start of the spinner culture, a part of the culture solution was sampled to measure the number of cells, the culture solution was adjusted to 1.0 × 10$^6$ cells/ml in a culture medium consisting of 25 $\mu$g/ml Gentamaicin, 16.25 $\mu$g/ml XAV939, 10% fetal bovine serum, and DMEM low-glucose in terms of final concentration, and then spinner culture was continued. From 9 days to 13 days after the start of the spinner culture, the culture medium was changed every two days with the same culture medium excluding XAV939, and then spinner culture was continued.

14 days after the start of the spinner culture, it was confirmed that a part of cell masses derived from each of clones A, B, D, E, F, G, I, J, K, L, M, N, O, R, S, T, U, W, X, Y, Z, a, b, and c beat autonomously. On the other hand, the beating was not observed in the cell masses derived from clones C, H, P, Q, and V. A part of the culture solution was sampled to measure the number of cells. The number of cells finally obtained is shown in Table 1.

[Table 1]

| Clone name | Total number of cells (× 10$^6$ cells) |
|---|---|
| A | 143.5 |
| B | 534.7 |
| C | 4.9 |
| D | 24.8 |
| E | 573.5 |
| F | 212.9 |
| G | 334.2 |
| H | 8.4 |
| I | 315.4 |
| J | 173.1 |
| K | 94.6 |
| L | 555.2 |
| M | 285.6 |
| N | 46.6 |
| O | 20.6 |
| P | 446.7 |
| Q | 94.0 |
| R | 376.3 |
| S | 351.4 |
| T | 584.7 |
| U | 38.3 |
| V | 89.3 |
| w | 12.7 |
| X | 337.2 |

(continued)

| Clone name | Total number of cells ($\times 10^6$ cells) |
|---|---|
| Y | 100.5 |
| Z | 49.1 |
| a | 176.0 |
| b | 619.6 |
| c | 462.7 |

Cell masses 14 days after the start of the spinner culture were separated into single cells by TrypLE Select, and dead cells were stained using a Live/Dead Fixable Green Dead Cell Stain Kit. After washing with D-PBS (Thermo Fisher Scientific, Inc.), the cells were fixed by treatment with formaldehyde (Sigma-Aldrich Co., LLC) of a final concentration of 4%. An anti-cardiac Troponin T (cTnT) antibody (Abcam plc, ab8295) was diluted to 1/250 in the D-PBS containing 0.1% Saponin and 2% fetal bovine serum in terms of final concentration and added to $0.5 \times 10^6$ cells of the obtained fixed cells, and the treatment was carried out at room temperature for 1 hour. At the same time, as an isotype control, a sample to which IgG1 isotype Control Murine Myeloma (Sigma-Aldrich Co., LLC, M5284) diluted to 1/25 had been added were paralleled. Subsequently, Alexa647-labeled Goat anti-mouse IgG1 antibody (Thermo Fisher Scientific, Inc., A21240) was diluted to 1/500 and added, and the treatment was carried out at room temperature for 30 minutes. The obtained labeled cells were analyzed using a flow cytometer. After gating forward light scattering, lateral light scattering, and live cells, the cTnT positive cell rate was calculated by comparison with the Isotype sample. Table 2 shows the cTnT positive rate of each clone and the number of cTnT positive cells (total number of myocardial cells) calculated from the total number of cells and the cTnT positive rate.

[Table 2]

| Clone name | cTnT positive rate of cells (%) | Total number of myocardial cells ($\times 10^6$ cells) |
|---|---|---|
| A | 42.7 | 61.3 |
| B | 14.4 | 77.0 |
| C | 0.1 | 0.0 |
| D | 22.8 | 5.6 |
| E | 25.5 | 146.2 |
| F | 16.8 | 35.8 |
| G | 62.2 | 207.9 |
| H | 1.1 | 0.1 |
| I | 44.9 | 141.6 |
| J | 41.6 | 72.0 |
| K | 40.1 | 37.9 |
| L | 11.6 | 64.4 |
| M | 41.8 | 119.4 |
| N | 16.9 | 7.9 |
| O | 5.0 | 10.8 |
| P | 0.4 | 1.7 |
| Q | 34.6 | 32.5 |
| R | 9.9 | 37.3 |
| S | 27.5 | 96.6 |
| T | 10.2 | 59.6 |

(continued)

| Clone name | cTnT positive rate of cells (%) | Total number of myocardial cells ($\times 10^6$ cells) |
|---|---|---|
| U | 1.2 | 0.5 |
| V | 20.5 | 18.3 |
| w | 3.6 | 0.5 |
| X | 9.3 | 31.3 |
| Y | 4.1 | 4.2 |
| Z | 3.1 | 1.5 |
| a | 29.2 | 51.4 |
| b | 18.7 | 115.9 |
| c | 5.1 | 23.4 |

For each of the 29 clones, a proportion of the total number of myocardial cells to the total number of cells at a timing of 14 days after the start of the spinner culture was derived as the differentiation rate E. Subsequently, the index value $\gamma$ indicating the complexity of the contour line of the colony and the differentiation rate E, acquired for every clone, were plotted on a graph, whereby the graph showing the relationship between the index value $\gamma$ indicating the complexity of the contour line of the colony and the differentiation rate E, shown in Fig. 9, was created. Further, when the relationship between the index value $\gamma$ indicating the complexity of the contour line of the colony and the differentiation rate E was mathematically expressed using a known function fitting method, a relationship expression of Expression (8) could be derived. The coefficient of determination $R^2$ in the function (the regression model) represented by Expression (8) was 0.7888, and the correlation coefficient R is 0.8881.

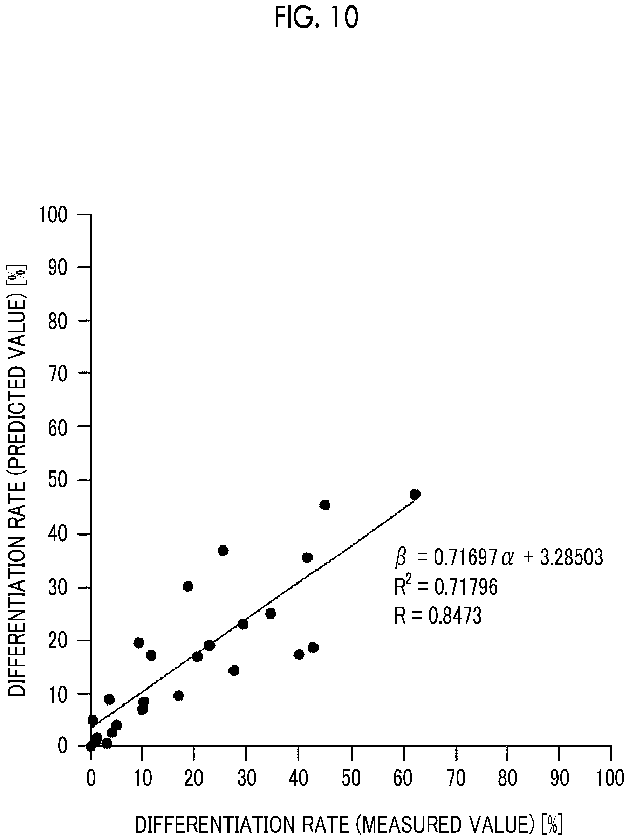

Fig. 10 is a graph showing a relationship between a predicted value (vertical axis) of the differentiation rate E calculated using Expression (8) and the measured value (horizontal axis) for each of the plurality of clones. The predicted value and the measured value of the differentiation rate, shown by each plot in Fig. 10, are intra-lot average values. The solid line in Fig. 10 is an approximate straight line derived based on each plot. In a case where the horizontal axis of the graph shown in Fig. 10 is denoted by $\alpha$ and the vertical axis thereof is denoted by $\beta$, the approximate straight line is expressed as $\beta = 0.71697\alpha + 3.28503$. The coefficient of determination $R^2$ in this approximate straight line is 0.71796, and the correlation coefficient R is 0.8473. That is, it can be said that the accuracy of the predicted value of the differentiation rate derived by using the function represented by Expression (8) is extremely high.

[Comparative Example]

In each method according to the embodiment of the disclosed technology, the suitability of an image other than the phase contrast image was also verified as the image for deriving the index value indicating the complexity or simplicity of the contour line of the colony. That is, as Comparative Example, the relationship between the complexity of the contour line of the colony derived based on an image of this colony (hereinafter referred to as a phase contrast microscopic image), the image being captured using a phase contrast microscope, and the differentiation rate E in a case where the pluripotent stem cells contained in this colony had been subjected to the differentiation induction treatment was examined.

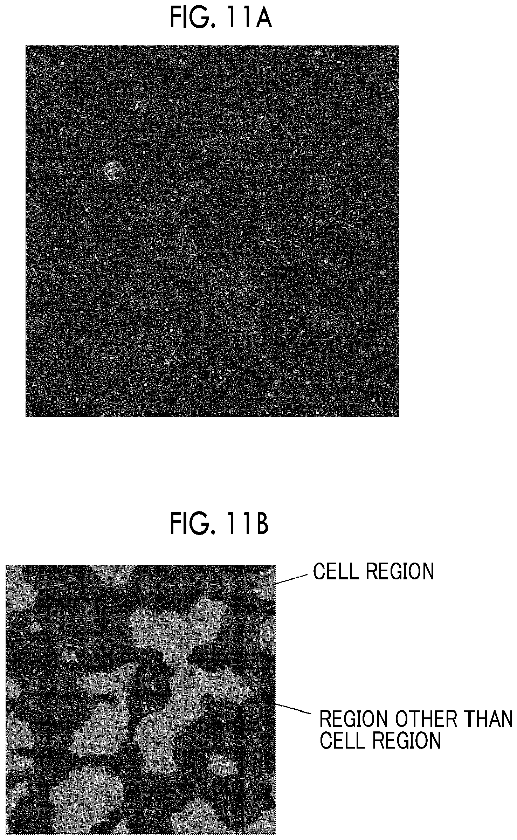

Fig. 11A is a phase contrast microscopic image of a colony of iPS cells adhered on a base material at the same timing as the sampling of iPS cells carried out in Example, that is, after expansion culture and before spinner culture. In this phase contrast microscopic image of colonies, a cell region and a region other than the cell region were specified by using semantic segmentation by deep learning. The cell region is a region in which all elements that constitute a cell, such as a cell nucleus, cytoplasm, and cell membrane, are shown in the phase contrast microscopic image. The region other than the cell region is the region that shows a region that is not occupied by the target cells but occupied by dead cells, debris, a culture medium, and the like in the phase contrast microscopic image. Fig.11B shows the specified cell region and region other than the cell region.

The contour lines of the specified cell regions were extracted, and Expression (6) was used to derive the index value $\gamma$ indicating complexity of the contour line of the colony based on the total A of areas of the cell regions surrounded by the contour lines and the total P of lengths of the contour lines.

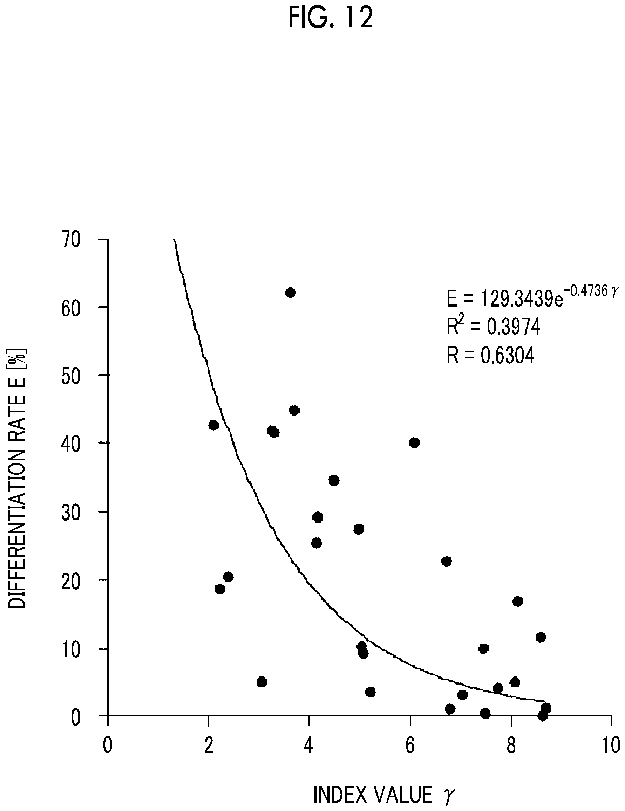

Fig. 12 is a graph showing one example of the relationship between the index value $\gamma$ which indicates the complexity of the contour line of the colony of iPS cells and derived from the phase contrast microscopic image, and the differentiation rate E of the myocardial cell which is obtained by the differentiation induction treatment on the iPS cells contained in this colony. Each plot shown in Fig. 12 corresponds to each of a plurality of production lots in the production process of the cell product, and the index value which indicates the complexity of the contour line of the colony and is indicated by each plot is the average value of the index values $\gamma$ which indicates the complexity of the contour line of the colony and has been acquired in regard to a plurality of colonies in the production lot.

For example, Expression (12) can be derived as a function that shows a relationship between the index value $\gamma$ indicating the complexity of the contour line of the colony and the differentiation rate E, shown in Fig. 12. The coefficient of determination $R^2$ in the function (the regression model) represented by Expression (12) is 0.3974, and the correlation coefficient R is 0.6304.

$$E = 129.3439\,e^{-0.4736\gamma} \quad \cdots (12)$$

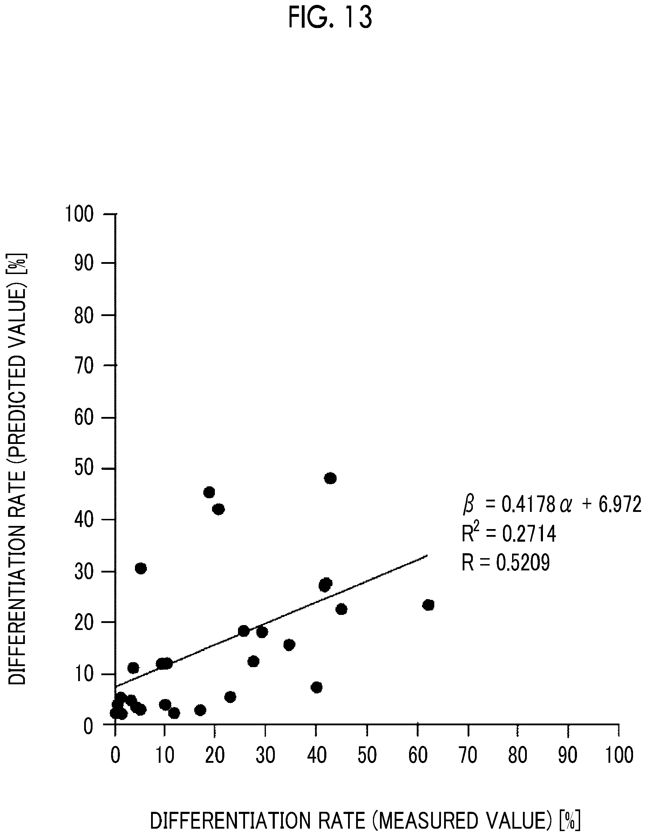

Fig. 13 is a graph showing a relationship between a predicted value (vertical axis) of the differentiation rate E calculated using Expression (12) and the measured value (horizontal axis) for each of the plurality of production lots. The predicted value and the measured value of the differentiation rate, shown by each plot in Fig. 13, are intra-lot average values. The solid line in Fig. 13 is an approximate straight line derived based on each plot. In a case where the horizontal axis of the graph shown in Fig. 13 is denoted by $\alpha$ and the vertical axis thereof is denoted by $\beta$, the approximate straight line is expressed as $\beta = 0.4178\alpha + 6.972$. The coefficient of determination $R^2$ in this approximate straight line is 0.2714, and the correlation coefficient R is 0.5209, which are significantly low as compared with the case (see Fig. 12) of using the phase contrast image that is generated from the hologram as an image that is used for deriving the index value $\gamma$ indicating the complexity of the contour line of the colony. That is, in a case where the phase contrast microscopic image is used as an image that is used for deriving the index value indicating the complexity or simplicity of the contour line of a colony, the accuracy of the predicted value of the differentiation rate E is significantly reduced as compared with the case where the phase contrast image that is generated from the hologram is used. In other words, in a case where the phase contrast image that is generated from the hologram is used as an image that is used for deriving the index value indicating the complexity or simplicity of the contour line of the colony, an index value having a high correlation with the differentiation rate E can be obtained, and thus the accuracy of the predicted value of the differentiation rate E can be improved.

The disclosure of JP2019-195671 filed on October 28, 2019, is incorporated in the present specification in its entirety by reference. In addition, all documents, patent applications, and technical standards described in the present specification are incorporated in the present specification by reference, to the same extent as in the case where each of the documents, patent applications, and technical standards is specifically and individually described.

**Claims**

1. A sorting method for a pluripotent stem cell, comprising:

    generating a phase contrast image of an aggregate of a pluripotent stem cell from a hologram in which the aggregate is captured;
    deriving, based on the phase contrast image, an index value indicating a complexity or simplicity of a contour line, the index value being determined according to a relationship between an area of the aggregate and a length of the contour line of the aggregate; and
    sorting the pluripotent stem cell based on the index value.

2. The sorting method according to claim 1,

    wherein the index value is a value that increases as the complexity of the contour line increases, and
    a pluripotent stem cell contained in an aggregate having the index value smaller than a predetermined threshold value is targeted for a differentiation induction treatment for differentiation into a specific cell.

3. The sorting method according to claim 1,

    wherein the index value is a value that increases as the simplicity of the contour line increases, and
    a pluripotent stem cell contained in an aggregate having the index value larger than a predetermined threshold value is targeted for a differentiation induction treatment for differentiation into a specific cell.

**4.** The sorting method according to claim 2 or 3,
wherein the threshold value is determined based on a relationship between the index value derived in regard to the aggregate before carrying out the differentiation induction treatment and a differentiation rate indicating a proportion of the number of the specific cells with respect to all cells at a time after carrying out the differentiation induction treatment on the pluripotent stem cell.

**5.** A prediction method for a differentiation induction result, comprising:

generating a phase contrast image of an aggregate of a pluripotent stem cell from a hologram in which the aggregate is captured;
deriving, based on the phase contrast image, an index value indicating a complexity or simplicity of a contour line, the index value being determined according to a relationship between an area of the aggregate and a length of the contour line of the aggregate; and
deriving, based on the index value, a predicted value regarding the number of specific cells obtained by carrying out a differentiation induction treatment for differentiating the pluripotent stem cell into the specific cell.

**6.** The prediction method according to claim 5,
wherein the predicted value is a differentiation rate indicating a proportion of the number of the specific cells with respect to all cells at a time after carrying out the differentiation induction treatment on the pluripotent stem cell.

**7.** The prediction method according to claim 6,
wherein a function that shows a relationship between the index value and the differentiation rate is used to derive the predicted value.

**8.** A production method for a cell product, comprising:

a culture step of culturing a pluripotent stem cell;
a sorting step of sorting the pluripotent stem cell cultured in the culture step; and
a differentiation induction step of carrying out a differentiation induction treatment for differentiating the pluripotent stem cell sorted in the sorting step into a specific cell,
wherein in the sorting step,
a phase contrast image of an aggregate of the pluripotent stem cell is generated from a hologram in which the aggregate is captured,
an index value indicating a complexity or simplicity of a contour line is derived based on the phase contrast image, where the index value is determined according to a relationship between an area of the aggregate and a length of the contour line of the aggregate, and
the pluripotent stem cell is sorted based on the index value.

**9.** The method according to any one of claims 2 to 8,
wherein the phase contrast image is generated from the hologram of the aggregate of the pluripotent stem cell in a state of being adhered on a base material, which is captured before carrying out the differentiation induction treatment.

**10.** The method according to any one of claims 2 to 9,
wherein in the phase contrast image, in a case where a threshold value is set, a first region having a phase contrast amount larger than the threshold value is regarded as a cell region in which cells are present, and a second region having a phase contrast amount smaller than the threshold value is regarded as a background, a total A of areas of the first region surrounded by a boundary line between the first region and the second region is derived as the area of the aggregate, and a total P of lengths of the boundary line is derived as the length of the contour line of the aggregate.

**11.** The method according to claim 10,
wherein $\gamma$ given according to Expression (I) is derived as the index value indicating the complexity of the contour line,

$$\gamma = P/2(\pi A)^{1/2} \quad \cdots \text{(I)}$$

**12.** The method according to claim 10,

wherein ε given according to Expression (II) is derived as the index value indicating the complexity of the contour line,

$$\varepsilon = P^2/4\pi A \quad \cdots \text{(II)}$$

13. The method according to claim 10,
    wherein p given according to Expression (III) is derived as the index value indicating the simplicity of the contour line,

$$\rho = 2(\pi A)^{1/2}/P \quad \cdots \text{(III)}$$

14. The method according to claim 10,
    wherein c given according to Expression (IV) is derived as the index value indicating the simplicity of the contour line,

$$c = 4\pi A/P^2 \quad \cdots \text{(IV)}$$

15. The method according to any one of claims 2 to 14, wherein the specific cell is a myocardial cell.

## FIG. 1

| | |
|---|---|
| EXPANSION CULTURE STEP | ~S1 |
| SORTING STEP | ~S2 |
| THREE-DIMENSIONAL CULTURE STEP | ~S3 |
| DIFFERENTIATION INDUCTION STEP | ~S4 |

## FIG. 2

| | |
|---|---|
| PHASE CONTRAST IMAGE IS GENERATED FROM HOLOGRAM IN WHICH COLONIES ARE CAPTURED. | ~S11 |
| INDEX VALUE INDICATING COMPLEXITY OF CONTOUR LINE OF COLONY IS DERIVED | ~S12 |
| PLURIPOTENT STEM CELLS ARE SELECTED BASED ON INDEX VALUE | ~S13 |

# FIG. 3

FIG. 4A

FIG. 4B

## FIG. 4C

## FIG. 4D

# FIG. 5

# FIG. 6

STATE OF BEING OUT OF FOCUS ——→

STATE OF BEING IN FOCUS ——→

——— STATE OF BEING IN FOCUS

- - - - - STATE OF BEING OUT OF FOCUS

PHASE CONTRAST AMOUNT

POSITION

PHASE CONTRAST AMOUNT

W

FREQUENCY

EP 4 036 556 A1

# FIG. 7

FIG. 8A

FIG. 8B

REGION WHERE CELLS
ARE NOT PRESENT
(FIRST REGION)

REGION WHERE CELLS
ARE PRESENT
(SECOND REGION)

FIG. 8C

# FIG. 9

$$E = 189.9504e^{-0.1931\gamma}$$
$$R^2 = 0.7888$$
$$R = 0.8881$$

# FIG. 10

$\beta = 0.71697\alpha + 3.28503$
$R^2 = 0.71796$
$R = 0.8473$

FIG. 11A

FIG. 11B

CELL REGION

REGION OTHER THAN
CELL REGION

# FIG. 12

$E = 129.3439e^{-0.4736\,\gamma}$
$R^2 = 0.3974$
$R = 0.6304$

# FIG. 13

$\beta$ = 0.4178 $\alpha$ + 6.972
$R^2$ = 0.2714
R = 0.5209

## INTERNATIONAL SEARCH REPORT

| | International application No. |
|---|---|
| | PCT/JP2020/037591 |

**A. CLASSIFICATION OF SUBJECT MATTER**
Int.Cl. G01N21/17(2006.01)i, C12N5/071(2010.01)i, C12Q1/04(2006.01)i
FI: C12Q1/04, C12N5/071, G01N21/17A

According to International Patent Classification (IPC) or to both national classification and IPC

**B. FIELDS SEARCHED**

Minimum documentation searched (classification system followed by classification symbols)
Int.Cl. G01N21/17, C12N5/071, C12Q1/04

Documentation searched other than minimum documentation to the extent that such documents are included in the fields searched
Published examined utility model applications of Japan       1922–1996
Published unexamined utility model applications of Japan     1971–2020
Registered utility model specifications of Japan             1996–2020
Published registered utility model applications of Japan     1994–2020

Electronic data base consulted during the international search (name of data base and, where practicable, search terms used)
JSTPlus/JMEDPlus/JST7580(JDreamIII),
CAplus/MEDLINE/EMBASE/BIOSIS/WPIDS(STN)

**C. DOCUMENTS CONSIDERED TO BE RELEVANT**

| Category* | Citation of document, with indication, where appropriate, of the relevant passages | Relevant to claim No. |
|---|---|---|
| Y<br>A | WO 2011/161962 A1 (KAWASAKI HEAVY IND LTD.) 29 December 2011 (2011-12-29), claims, paragraphs [0008], [0032]-[0052] | 1-4, 8-15<br>5-7 |
| Y<br>A | WO 2019/176427 A1 (FUJIFILM CORPORATION) 19 September 2019 (2019-09-19), claims, paragraphs [0014], [0043]-[0049] | 1-4, 8-15<br>5-7 |
| Y<br><br>A | WO 2018/189877 A1 (HITACHI HIGH-TECHNOLOGIES CORP.) 18 October 2018 (2018-10-18), claims, paragraphs [0043], [0044] | 1, 2, 4, 8-12, 15<br>3, 5-7, 13, 14 |
| Y<br><br>A | JP 2007-24612 A (PANASONIC ECOLOGY SYSTEMS CO., LTD.) 01 February 2007 (2007-02-01), claims, paragraphs [0047], [0048] | 1, 2, 4, 8-12, 15<br>3, 5-7, 13, 14 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 12 November 2020 | 24 November 2020 |

| Name and mailing address of the ISA/<br>Japan Patent Office<br>3-4-3, Kasumigaseki, Chiyoda-ku,<br>Tokyo 100-8915, Japan | Authorized officer<br><br><br>Telephone No. |
|---|---|

Form PCT/ISA/210 (second sheet) (January 2015)

INTERNATIONAL SEARCH REPORT

Information on patent family members

| International application No. |
| --- |
| PCT/JP2020/037591 |

```
WO 2011/161962 A1  29 December 2011    US 2013/0130228 A1
                                       claims, paragraphs [0043]-[0073]
                                       EP 2586872 A1

WO 2019/176427 A1  19 September 2019   (Family: none)

WO 2018/189877 A1  18 October 2018     US 2020/0056141 A1
                                       claims, paragraphs [0053]-[0056]

JP 2007-24612 A    01 February 2007    (Family: none)
```

Form PCT/ISA/210 (patent family annex) (January 2015)

**EP 4 036 556 A1**

REFERENCES CITED IN THE DESCRIPTION

**Patent documents cited in the description**

- JP 2015146747 A
- JP 2018000048 A
- WO 2018158901 A
- JP 5984217 B
- JP 2019195671 A